(19) Europäisches Patentamt
European Patent Office
Office européen des brevets

(11) **EP 2 209 462 B1**

(12) **EUROPÄISCHE PATENTSCHRIFT**

(45) Veröffentlichungstag und Bekanntmachung des Hinweises auf die Patenterteilung:
**10.04.2013 Patentblatt 2013/15**

(21) Anmeldenummer: **08849039.6**

(22) Anmeldetag: **14.11.2008**

(51) Int Cl.:
***A61K 8/97*** *(2006.01)*　　***A61Q 17/00*** *(2006.01)*

(86) Internationale Anmeldenummer:
**PCT/EP2008/065594**

(87) Internationale Veröffentlichungsnummer:
**WO 2009/063068 (22.05.2009 Gazette 2009/21)**

(54) **Kosmetisches Produkt zum Schutz der Haut gegen Umwelteinflüsse**

Cosmetic product for protecting the skin from environmental influences

Produit cosmétique permettant de protéger la peau contre les effets de la pollution environnementale

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **14.11.2007 DE 102007055008**

(43) Veröffentlichungstag der Anmeldung:
**28.07.2010 Patentblatt 2010/30**

(73) Patentinhaber: **COTY GERMANY GMBH 55116 Mainz (DE)**

(72) Erfinder:
• **GOLZ-BERNER, Karin**
**MC-98000 Monaco (MC)**
• **ZASTROW, Leonhard**
**MC-98000 Monaco (MC)**

(74) Vertreter: **Gulde Hengelhaupt Ziebig & Schneider Patentanwälte - Rechtsanwälte Wallstrasse 58/59 10179 Berlin (DE)**

(56) Entgegenhaltungen:
EP-A- 0 968 709　　WO-A-00/64472
WO-A-2006/094551　　WO-A-2006/111666
DE-A1-102004 045 411　　FR-A- 2 768 925
FR-A- 2 831 444　　US-A1- 2007 003 536

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 2 209 462 B1

**Beschreibung**

Die Erfindung betrifft ein kosmetisches Produkt zum Schutz der Haut gegen Umwelteinflüsse, insbesondere zum Schutz gegen verschmutzte Luft und /oder Elektrosmog. Unter verschmutzter Luft wird im Sinne der Erfindung eine Veränderung der natürlichen Zusammensetzung der Luft durch Rauch, Ruß, Staub, Gase, Aerosole, Dämpfe und/oder Geruchsstoffe verstanden. Unter Elektrosmog werden technisch verursachte elektrische, magnetische und/oder elektromagnetische Felder verstanden. Das erfindungsgemäße kosmetische Produkt schützt die Haut insbesondere gegen Rauch, Ruß und/oder Staubpartikel, ganz besonders gegen Partikel aus Zigarettenrauch und/oder Dieselruß.

Es sind bereits eine Reihe von Vorschlägen gemacht worden, Kosmetika für Haut und Haare gegen diese nachteiligen Umwelteinflüsse einzusetzen. Als wirksame Bestandteile sind dabei beispielsweise in der US 5,683,684 ein Sphingolipid zusammen mit einem Chelatisierungsmittel eingesetzt worden. In der US 2004/047823 wird Ergothionein oder ein Derivat davon vorgeschlagen und in der US 2004/013696 Ellagsäure als Wirksubstanz. Auch Aminosäuren (US 2002/0168328) sowie eine Reihe von Pflanzenextrakten wie Pinus lambertania (WO 00/59465), Lampsana communis und Camellia sinensis (WO 2006/111666) sowie Theobroma cacao (EP 1498113) sind als kosmetisch wirkend genannt worden.

FR-A-2 768 925 offenbart kosmetische Produkte, die einen Hedera Helix Extrakt enthalten. FR-A-2 831 444 offenbart kosmetische Produkte, die Extrakte aus Buddleja davidii und Thymus vulgaris enthalten. EP-A-968 709 offenbart kosmetische Produkte, die Extrakte aus Camelia siensis, thymus vulgaris und Ginkgo biloba enthalten. US-A1-2007/003536 offenbart kosmetische Produkte, die Extrakte aus Citrus grandis und Camelia sienensis enthalten.

Aufgabe der vorliegenden Erfindung ist die Bereitstellung eines kosmetischen Produktes, das eine sehr breite und langanhaltende Wirksamkeit gegen die genannten nachteiligen Umwelteinflüsse auf die Haut zeigt.

Eine weitere Aufgabe besteht darin, eine frühzeitige Hautalterung infolge des Einflusses von Rauch, insbesondere Zigarettenrauch, deutlich einzuschränken.

Eine weitere Aufgabe besteht darin, die Spannung in der Epidermis zu verbessern und die Haut, insbesondere Umwelteinflüssen besonders ausgesetzte Haut von Gesicht, Hals, Decolleté und Armen glänzender und frischer erscheinen zu lassen.

Dabei soll die Verbesserungen nicht über eine Filmbildung auf der Haut erreicht werden, sondern über eine direkt deaktivierende Wirkung auf die Umweltgifte.

Die Aufgabe der Erfindung wird durch die Bereitstellung eines kosmetischen Produktes gelöst, das folgende Bestandteile enthält:

- ein Pflanzenextraktgemisch, bestehend aus Hedera helix-Extrakt, Buddleja davidii-Extrakt, Thymus vulgaris-Extrakt, Ginkgo biloba-Extrakt, Camellia sinensis-Extrakt und Citrus grandis-Extrakt;
- einen Sauerstoffträger, der aus einem flüssigen perfluorierten oder teilfluorierten Kohlenwasserstoff oder Kohlenwasserstoffgemisch, einem flüssigen Siliconpolymeren oder Siliconpolymerengemisch und einer Öl- oder Wasserbasis besteht, wobei dieses Trägersystem mit gasförmigem Sauerstoff beladen ist;
- einen Radikalfänger, der einen Komplex darstellt, bestehend aus Wasser, Gelbildner, Phospholipiden, Seidenraupen-Extrakt und einem durch Extraktion der Rinde von Quebracho blanco und nachfolgende enzymatische Hydrolyse gewonnenem Produkt oder der ein Gemisch von Pflanzenextrakten auf alkoholischer Basis darstellt, bestehend aus Angelica archangelica-Wurzelextrakt, Pongamia pinnata-Samenextrakt, Camellia sinensis-Blattextrakt, Coffea arabica-Samenextrakt, Ethanol im Verhältnis 0,2 : 0,2 : 0,2 : 0,2 : 99,2;
- ein teilchenförmiges, anorganisches oder organisches kosmetisch annehmbares Material mit einer mittleren Teilchengröße im Bereich von 1-25 $\mu$m ;

und

kosmetische Hilfsstoffe, Trägerstoffe und Gemische davon.

Es wurde gefunden, dass die erfindungsgemäße Kombination von Pflanzenextrakten, Radikalfängern, Sauerstofflieferanten und teilchenförmigem Material eine außerordentlich gute Wirkung gegen schädigende Umwelteinflüsse zeigt. Anders als bei einer Reihe von bekannten technischen Lösungen erfolgt eine direkte Wirkung durch Beseitigung freier Radikale auf der Haut, ohne dass ein Filmbildner eine Abschirmungsfunktion übernimmt.

Messungen des Radikalschutzfaktors RPF haben gezeigt, dass das radikalfangende Potential der Haut bei Anwendung des erfindungsgemäßen kosmetischen Produktes weitgehend erhalten bleibt und nur geringfügig zurückgeht, wenn eine konzentrierte kurzzeitige Schädigung durch Umwelteinflüsse wie Dieselpartikel, Teer aus Zigarettenrauch usw. zu erwarten war.

Der Radikalschutzfaktor (Radical Protection Factor) bestimmt die Aktivität zur Neutralisierung freier Radikale durch ein Antioxidationsmittel gegenüber einer Testsubstanz. Diese Testsubstanz besteht aus einem sehr reaktionsfähigen, halbstabilen Radikal, das mit allen bekannten Antioxidationsmitteln reagiert. Die Messung des RPF erfolgt in der Weise, dass die Signalamplitude des Testradikals durch Elektronenspinresonanz (ESR/EPR) vor und nach dem Vermischen mit einem Antioxidationsmittel gemessen und daraus der RPF berechnet wird.

Das genaue Messverfahren ist beschrieben von Herrling, Groth, Fuchs und Zastrow in Conference Materials "Modern Challenges To The Cosmetic Formulation" 5.5.-7-5.97, Düsseldorf, S: 150-155, Verlag f. chem. Ind. 1997. Dabei wird, ausgehend von der bekannten Konzentration des Testradikals oder der Anzahl von dessen freien Radikalen (Radikale pro ml) eine Signalamplitude $S_1$ mittels eines ESR-Spektrometers gemessen. Das Testradikal ist ebenso wie das Antioxidationsmittel in einer Wasser/Alkohollösung gelöst. Dann wird die Signalamplitude $S_2$ des Antioxidationsmittels gemessen. Die normalisierte Differenz zwischen den beiden Signalamplituden ist der Reduktionsfaktor RF.

$$RF = (S_1 - S_2) / S_1$$

Das Ergebnis der Testradikalreduzierung RC x RF wird zu der Menge der Produkteingabe PI (mg/ml) normalisiert. Der Radikalschutzfaktor wird nach der folgenden Gleichung berechnet

$$RPF = \frac{RC(Radikale/ml) \times RF}{PI(mg/ml)}$$

Das Ergebnis ist RPF = N x $10^{14}$ [Radikale pro mg], wobei N eine positive reale Zahl ist. Bei der Angabe des RPF wird zur Vereinfachung oft nur die Zahl N ohne den Zusatz $10^{14}$ benutzt. Das Verfahren ist auch in der WO 99/66881 beschrieben.

Die erfindungsgemäß eingesetzten Pflanzenextrakte sind Blattextrakte, die bei Umgebungstemperatur (18-25 °C) mit Wasser, Propylenglycol, Glycerin, Propylenglycol/Wasser- oder Glycerin/Wasser- Gemischen gewonnen wurden. Der Extrakt von Camellia sinensis ist ein Extrakt von weißem Tee, d.h. von im frühen Blütestadium geernteten Teeblättern.

Als Hedera helix-Extrakt wird erfindungsgemäß bevorzugt der unter dem INCI-Namen Propylene Glycol, Water, Hedera Helix (Ivy) Extract bekannte Auszug eingesetzt (CAS No. 7732-18-5, 57-55-6, 84650-60-2). Der erfindungsgemäß bevorzugt eingesetzte Camellia sinensis-Extrakt ist unter dem INCI-Namen Water, Propylene Glycol, Camellia sinensis leaf extract bekannt (CAS No. 7732-18-5, 57-55-6, 84650-60-2). Die erfindungsgemäß eingesetzten Ginkgo-Extrakte sind als Ginkgo Biloba-Extrakte oder als Herbalia® Ginkgo (CAS No. 90045-36-6) bekannt. Der INCI-Name für den erfindungsgemäß bevorzugt eingesetzten Buddleja davidii- und Thymus vulgaris-Extrakt lautet Glycerin, Water, Buddleja davidii Extract (and) Thymus vulgaris (Thyme) Extract (CAS No. 56-81-5, 7732-18-5, 94465-67-5, 84929-51-1). Dieses Produkt wird auch unter dem Namen Nectapure beispielsweise von der Firma Pentapharm Ltd., Basel, Schweiz vertrieben.

Als Citrus grandis-Extrakt wird erfindungsgemäß bevorzugt der unter dem INCI-Namen Citrus grandis leaf extract bekannte Auszug eingesetzt (CAS No. 90045-43-5).

Bevorzugte Gehalte für die Pflanzenextrakte sind Ginkgo-Extrakt 0,5 - 2,5 Gew.-%; Buddleja davidii & Thymus vulgaris-Extrakt 0,01 - 3,0 Gew.-%, vorzugsweise 0,01 - 1,0 Gew.-%; Hedera helix-Extrakt 0,01 - 3,0 Gew.-%, vorzugsweise 0,2 - 1,8 Gew.-%; Camellia sinensis-Extrakt 0,1 - 1,0 Gew.-%, vorzugsweise 0,2 - 0,8 Gew.-%; Citrus grandis-Extrakt 0,1 - 2,0 Gew.-%, vorzugsweise 0,1 - 0,7 Gew.-%.

Der Gesamtgehalt der Pflanzenextrakte liegt nicht über 5 Gew.-%.

Als Radikalfänger kann eine Wirkstoffzubereitung mit hohem Radikalschutzfaktor eingesetzt werden mit einem Gehalt an einem durch Extraktion der Rinde von Quebracho blanco und nachfolgender enzymatischer Hydrolyse gewonnenem Produkt, das wenigstens 90 Gew.-% Proanthocyanidin-Oligomere und höchstens 10 Gew.-% Gallussäure enthält, in Mikrokapseln, sowie einem durch Extraktion gewonnenen Seidenraupenextrakt, der das Peptid Cecropine, Aminosäuren und ein Vitamingemisch enthält, und einem nichtionischen, kationischen oder anionischen Hydro-Gel oder Gemisch von Hydro-Gelen, und einem oder mehreren Phospholipiden, und Wasser, z.B. ein Produkt gemäß Anspruch 1 der WO 99/66881 , insbesondere der Wirkstoffkomplex gemäß Beispiel 1 oder 2, oder ein Wirkstoffkomplex gemäß Anspruch 1 der WO 01/26617, insbesondere der Wirkstoffkomplex gemäß Beispiel 1.

Der Gehalt für diesen Wirkstoffkomplex mit hohem Radikalschutzfaktor liegt vorzugsweise im Bereich von 0,3 - 1,5 Gew.-%.

Als Radikalfänger kann eine alkoholische Wirkstoffzubereitung gemäß Anspruch 1 der WO 2004/105704 eingesetet werden, die ein liposomenfreies Gemisch von Pflanzenextrakten auf alkoholischer Basis darstellt, bestehend aus bestehend aus Angelica archangelica-Wurzelextrakt, Pongamia pinnata-Samenextrakt, Camellia sinensis-Blattextrakt, Coffea arabica-Samenextrakt, Ethanol im Verhältnis 0,2 : 0,2 : 0,2 : 0,2 : 99,2.

Die Zugabemenge zu dem erfindungsgemäßen kosmetischen Produkt beträgt 0,1 - 2,0 Gew.-%.

Der weiterhin als Wirkstoff enthaltene Sauerstoffträger besteht aus einem flüssigen perfluorierten oder teilfluorierten Kohlenwasserstoff oder Kohlenwasserstoffgemisch, vorzugsweise mit einem Anteil von 0,1-10 Gew.-%, einem flüssigen Siliconpolymeren oder Siliconpolymerengemisch, vorzugsweise mit einem Anteil von 10-85 Gew.-%, einer Öl- oder Wasserbasis, vorzugsweise mit einem Anteil von 5-25 Gew.-%, wobei alle Gewichtsanteile auf das Gesamtgewicht des Trägersystems bezogen sind. Das Trägersystem ist mit gasförmigem Sauerstoff, vorzugsweise bis zu einem Partialdruck von 150-950 mbar $O_2$ ,beladen. Das Sauerstoff-Trägersystem liegt in dem kosmetischen Produkt in einem Anteil von 1-40 Gew.-% vor, bezogen auf das Gesamtgewicht des Produktes. Dieses Trägersystem hält den Sauerstoff deutlich länger als andere bekannte Systeme mit Peroxiden oder Phospholipiden, da es z.B. mit einem Sauerstoffgehalt vier Wochen nach der Beladung in Höhe von 25-40 Vol.-% des Anfangs-Sauerstoffgehaltes noch beladen ist.

Überraschenderweise wird diese Eigenschaft durch die vorhandenen Pflanzenextrakte nicht gestört ,sondern noch weiter verbessert.

Bevorzugte Gehalte des Sauerstoffträgers liegen bei 0,1 - 5,0 Gew.-%, vorzugsweise 1,0 - 4,0 Gew.-%.

Zu den in der Erfindung eingesetzten teilchenförmigen Materialien gehören vorzugsweise Pigmente, Pigmentgemische oder Pulver mit pigmentartiger Wirkung, zum Beispiel Eisenoxide, natürliche Aluminiumsilicate, Titandioxid, Siliciumdioxid, Calciumcarbonat, Bornitrid, Glimmer, Kaolin, Nylonkügelchen, Keramikkügelchen, expandierte und nichtexpandierte synthetische Polymerpulver, pulverförmige natürliche organische Verbindungen wie gemahlene Festalgen, gemahlene Pflanzenteile, verkapselte und unverkapselte Getreidestärken sowie Gemische davon. Besonders bevorzugt sind Eisenoxide, natürliche Aluminiumsilicate, Titandioxid, Siliciumdioxid und Gemische davon. Erfindungsgemäß bevorzugt wird ein Gemisch aus Siliciumdioxid, Titandioxid und Eisenoxiden eingesetzt. Ein solches Gemisch ist beispielsweise unter dem INCI-Namen Silica, Titanium Dioxide (für EU: CI77891), Iron Oxides (für EU: C177491) bekannt und enthält 74-85 Gew.-% Siliciumdioxid, 15-24 Gew.-% Titandioxid und weniger als 2 Gew.-% Eisenoxide.

Der Gehalt an teilchenförmigen Materialien liegt im Bereich von 0,2 bis 5,0 Gew.-%, vorzugsweise 1,2 bis 3,5 Gew.-%.

Die Teilchengröße liegt im Bereich kleiner 25 μm (wenigstens 80% der Teilchen in diesem Bereich), besonders bevorzugt im Bereich von 3 bis 20 μm. Insbesondere liegt die mittlere Teilchengröße $D_{50}$ im Bereich von 3-10 μm, besonders bevorzugt von 4-7 μm. Die Teilchengröße wird mittels eines MALVERN® 2000 gemessen.

Teilchengrößen außerhalb dieses Bereiches lassen sich entweder nicht vorteilhaft in die Emulsion einbeziehen oder unterstützen nicht optimal die angestrebten Eigenschaften von Glanz und Frische-Erscheinung der Haut.

Das erfindungsgemäße Produkt enthält weiterhin kosmetische Hilfs- und/oder Trägerstoffe, wie sie üblicherweise in solchen Zubereitungen verwendet werden, z.B. Wasser, Konservierungsmittel, Farbstoffe, Pigmente mit färbender Wirkung, Verdickungsmittel, Duftstoffe, Alkohole, Polyole, Ester, Elektrolyte, Gelbildner, polare und unpolare Öle, Polymere, Copolymere, Emulgatoren, Wachse, Stabilisatoren.

Darüber hinaus können auch solche Stoffe enthalten sein, wie anorganische und organische Lichtschutzmittel, Selbstbräunungsmittel, Feuchthaltemittel, Melanin, entzündungswidrige natürliche Wirkstoffe, Ubichinon Q10, Kreatin, Kreatinin, Camitin, Biotin, Isoflavone, Cardiolipin, Liponsäure, Anti Freezing Proteine, Arctiin, Hopfen- und Hopfen-Malz-Extrakte, Harnstoff und Mineralsalze, insbesondere NaCl, Meeresmineralien sowie Osmolyte, Phytoen, Phytofluen, ζ-Carotin, Neurosporin, Lycopin, β-Carotin, Squalan, Variabilin, Phytansäure und/oder Phytol. Vorzugsweise enthält das erfindungsgemäße Produkt keine Filmbildner, um ein direktes Eindringen und Speichern der Wirksubstanzen in die obersten Hautschichten zu ermöglichen und dort die entsprechende anti-radikalische Wirkung zu erzielen.

Die für die Erfindung eingesetzten Öle können übliche kosmetische Öle sein, wie ein Mineralöl; hydriertes Polyisobuten; synthetisches oder aus Naturprodukten hergestelltes Squalan; kosmetische Ester oder Ether, die verzweigt oder unverzweigt, gesättigt oder ungesättigt sein können; pflanzliche Öle; oder Gemische zweier oder mehrerer davon.

Besonders geeignete Öle sind beispielsweise Siliconöle wie Dimethicone, Cyclomethicone, Dimethiconol; Mineralöle; Hydrogenated Polyisobuten, Polyisopren, Squalan, Trimethylpropan-triisostearat, Isodecylcitrat, Neopentylglycol-diheptanoat, PPG-15-stearylether; sowie pflanzliche Öle, wie Calendulaöl, Jojobaöl, Avocadoöl, Macadamianussöl, Rizinusöl, Kakaobutter, Kokosnussöl, Maisöl, Baumwollsamenöl, Olivenöl, Palmkernöl, Rapssamenöl, Safloröl, Sesamsamenöl, Sojabohnenöl, Sonnenblumensamenöl, Weizenkeimöl, Traubenkernöl, Kukuinussöl, Distelöl und Gemische davon; sowie Gemische dieser Öle.

Als Ester oder Ether sind zum Beispiel geeignet Glyceryl Stearate, PEG-100 Stearate, Ceteareth-20, Propylene Glycol Dioctanoate, Propylene Glycol Dicaprylate-2,30-Dicaprate, Tridecyl Stearate/Neopentyl Glycol Dicaprylate, Dicaprate/Tridecyl Trimellitate, Neopentyl Glycol Dioctanoate, Isopropyl Myristate, Diisopropyl Dimer Dilinoleate, Trimethylpropane Triisostearate, Myristyl Ether, Stearyl Ether, Cetearyl Octanoate, Butyl Ether, Dicaprylyl Ether, PPG1-PEG9 Lauroyl Glycol Ether, PPG15 Stearyl Ether, PPG14 Butyl Ether, Fomblin HC25.

Es ist weiterhin vorteilhaft, dem erfindungsgemäßen Produkt entsprechende wasser - und/oder öllösliche UVA- oder UVB-Filter oder beide zuzusetzen. Zu vorteilhaften öllöslichen UVB-Filtern gehören 4-Aminobenzoesäure-Derivate wie der 4-(Dimethylamino)-benzoesäure-(2-ethylhexyl)ester; Ester der Zimtsäure wie der 4-Methoxyzimtsäure(2-ethylhexyl)ester; Benzophenon-Derivate wie 2-Hydroxy-4-methoxybenzophenon; 3-Benzylidencampher-Derivate wie 3-Benzylidencampher.

Bevorzugte öllösliche UV Filter sind Benzophenone-3, Butyl-Methoxybenzoyl-methane, Octyl Methoxycinnamate, Octyl Salicylate, 4-Methylbenzylidene Camphor, Homosalate und Octyl Dimethyl PABA.

Wasserlösliche UVB-Filter sind z.B. Sulfonsäurederivate von Benzophenon oder von 3-Benzylidencampher oder Salze wie das Na- oder K-Salz der 2-Phenyl-benzimidazol-5-sulfonsäure.

Zu UVA-Filtern gehören Dibenzoylmethan-Derivate wie 1-Phenyl-4-(4'-isopropylphenyl)propan-1,3-dion, Butyl Methoxybenzoyl-methane oder Menthyl Anthranilate.

Besonders bevorzugt sind Benzophenone-3, Butyl Methoxydibenzoylmethane, Octyl Methoxycinnamate, Octyl Salicylate, 4-Methylbenzylidene Camphor, Homosalate, Octocrylene, Ethylhexyl Methoxycinnamate, Isoamyl-p-Methoxycinnamate, Octyl Dimethyl PABA, Ethylhexyltriazone, Diethylhexyl Butamido Triazone, Ethylhexyl Salicylate, Methylene Bis-Benzotriazolyl Tetramethylbutylphenol, Disodium Phenyl Dibenzimidazole Tetrasulfonate, Bis-Ethylhexyloxyphenol Methoxyphenyl Triazine.

Weiterhin können auch Breitbandfilter eingesetzt werden, wie z.B. Bis-Resorcinyltriazin-Derivate, oder auch Benzoxazole.

Weiterhin einsetzbar als Sonnenschutzfilter sind anorganische Pigmente auf Basis von Metalloxiden, wie $TiO_2$, $SiO_2$, ZnO, $Fe_2O_3$, $ZrO_2$, MnO, $Al_2O_3$, die auch im Gemisch verwendet werden können.

Als Erweichungsmittel können normalerweise eine Vielzahl von Verbindungen eingesetzt werden, wie Stearylalkohol, Glycerylmonoricinoleat, Glycerylmonostearat, Propan-1,2-diol, Butan-1,3-diol, Cetylalkohol, Isopropylisostearat, Stearinsäure, Isobutylpalmitat, Isopropylmyristat, Isopropylpalmitat, Oleylalkohol, Isopropyllaurat, Decyloleat, Octadecan-2-ol, Isocetylalkohol, Cetylpalmitat, Siliconöle wie Dimethylpolysiloxan, Polyethylenglycol, Lanolin, Kakaobutter, pflanzliche Öle wie Maisöl, Baumwollsamenöl, Olivenöl, mineralische Öle, Butylmyristat, Palmitinsäure usw.

Mit dem erfindungsgemäßen kosmetischen Produkt wird eine sehr breite und langanhaltende Wirksamkeit gegen verschiedene nachteilige Umwelteinflüsse auf die Haut erreicht. In Vergleichsversuchen mit Dieselrußpartikeln und mit Teer aus Zigarettenrauch wurde dies bestätigt.

Durch die Erhöhung des antioxidativen Potentials der Haut wird auch eine frühzeitige Hautalterung infolge des Einflusses von Rauch, insbesondere Zigarettenrauch, deutlich eingeschränkt und damit eine Verringerung von kleinen Hautfalten.

Insgesamt tragen die kombinierten Wirkstoffe auch zu einer Verbesserung der Spannung in der Epidermis bei, was in Dehnungstests bestätigt wurde. Besonders den Umwelteinflüssen stärker ausgesetzte Hautpartien von Gesicht, Hals, Decolleté und Armen sehen nach der Behandlung mit dem erfindungsgemäßen Produkt glänzender und frischer aus. Anwendertests bestätigen diese Aussage.

Als besonderer Vorteil der Erfindung gegenüber anderen bekannten technischen Lösungen wird angesehen, dass die Verbesserungen nicht über eine Filmbildung auf der Haut erreicht werden, sondern über eine direkt deaktivierende Wirkung der Umweltgifte in den oberen Hautschichten.

Die Verwendung der erfindungsgemäßen kosmetischen Zusammensetzungen kann z.B. erfolgen in Form von Sonnencremes, Sonnengelen, After-sun-Produkten, Tagescremes, Nachtcremes, Masken, Körperlotionen, Reinigungsmilch, Körperpuder, Augenkosmetik, Haarmasken, Haarspülungen, Haarshampoos, Duschgelen, Duschölen, Badeölen und in Produkten der dekorativen Kosmetik wie Deo-Stiften, Parfüm-Stiften, Lippenstiften, Lippenbalsam, Gelen, Lidschatten, Kompaktprodukten wie Kompaktpuder oder Kompaktwachs, Rouge, Grundierung, Make-up usw.

Die Herstellung derartiger Produkte erfolgt auf eine Weise, wie sie dem Fachmann auf diesem Gebiet bekannt ist.

Die Erfindung betrifft auch die Verwendung des oben genannten kosmetischen Produktes gegen Umwelteinflüsse aus der Luft, insbesondere gegen Zigarettenrauch, Dieselrußpartikel und Gemische davon.

Die Erfindung soll nachstehend durch Beispiele näher erläutert werden. Alle Angaben erfolgen in Gewichtsprozent, sofern nichts anderes angegeben ist.

Beispiel 1 Tagescreme mit SPF 10

| Phase A | |
| --- | --- |
| Wasser | q.s. ad 100 |
| Glycerine | 2,0 |
| Propylene Glycole | 3,0 |
| Citric Acid | 0,08 |
| Ethylene Diamine Tetra Acetic Acid | 0,1 |

(fortgesetzt)

| | |
|---|---|
| Phase B | |
| Glyceryl Stearate | 3,5 |
| PEG-100 Stearate | 1,0 |
| Ceteareth - 20 | 3,5 |
| Isoamyl-Methoxycinnamate | 4,0 |
| Butyl Methoxydibenzoylmethane | 2,0 |
| Phase C | |
| Silicone Oil (Dimethicone) | 3,0 |
| Phase D | |
| Parfümöl | 0,2 |
| Konservierungsmittel | 0,5 |
| RPF-Komplex 1* | 0,8 |
| SiOx** | 3,5 |
| Weißer Tee-Extrakt | 0,5 |
| Ginkgo-Extrakt | 1,5 |
| Buddleja davidii & Thymus vulgaris Extrakt | 0,01 |
| Citrus grandis Extrakt | 0,6 |
| Efeu-Extrakt | 0,5 |
| Sheabutter | 0,5 |
| Silica & Titanium Dioxide & Iron Oxides | 1,5 |

*RPF-Komplex 1 gemäß Anspruch 1 der WO 99/66881 (mit folgenden Gewichtsanteilen, bezogen auf das Gesamtgewicht des Komplexes): 1 % Gelbildner, 7,5 % Phospholipide, 2 % Quebracho-Extrakt ,1 % Seidenraupen-Extrakt, Wasser.

** SiOx:Trägersystem bestehend aus Dimethicone 20%, Trifluoromethyl C1-4 Alkyl Dimethicone 35%, Perfluorodecalin 5%, Dimethicone/Vinyldimethicone Crosspolymer (and) Cl2-14 Pareth-12 25%, Wasser 15%. Das bei 20-25°C hergestellte Gemisch wird mit gasförmigem Sauerstoff mit 400 mbar für 30 Minuten unter Rühren durchperlt. Der erhaltene Sauerstoffgehalt von 155 mbar $O_2$ wurde mit einem Oxi 3000 (WTW GmbH, Weilheim, Deutschland) gemessen.

Die Phasen A und B wurden separat auf 80°C erhitzt und dann unter Rühren zusammengegeben. Das Gemisch wurde auf 45°C abgekühlt, und die Phase C wurde unter Rühren hinzugeben. Danach erfolgte eine Abkühlung auf 35°C und die Bestandteile der Phase D wurden nacheinander homogen eingerührt.

Beispiel 2 Tagescreme

| | |
|---|---|
| Phase A | |
| Wasser | q.s. ad 100 |
| Glycerine | 2,0 |
| Propylene Glycole | 3,0 |
| Citric Acid | 0,08 |
| Ethylene Diamine Tetra Acetic Acid | 0,1 |
| Phase B | |
| Glyceryl Stearate | 3,5 |
| PEG-100 Stearate | 1,0 |
| Ceteareth - 20 | 3,5 |
| Phase C | |
| Silicone Oil (Dimethicone) | 3,0 |

(fortgesetzt)

| Phase D | |
|---|---|
| Parfümöl | 0,2 |
| Konservierungsmittel | 0,5 |
| RPF-Komplex 1* | 0,8 |
| SiOx** | 3,5 |
| Weißer Tee-Extrakt | 0,5 |
| Ginkgo-Extrakt | 1,5 |
| Buddleja davidii & Thymus vulgaris Extrakt | 0,01 |
| Citrus grandis Extrakt | 0,5 |
| Efeu-Extrakt | 0,5 |
| Sheabutter | 0,5 |
| Silica & Silicium Dioxide & Iron Oxide | 1,2 |
| * und ** siehe Beispiel 1 | |

Beispiel 3 Gel

| Phase A | |
|---|---|
| Wasser | q.s. ad 100 |
| Glycerine | 2,0 |
| Propylene Glycole | 3,0 |
| Carbomer | 2,0 |
| **Phase B** | |
| Triethanolamin | 2,0 |
| **Phase C** | |
| RPF-Komplex 3*** | 2,0 |
| Konservierungsmittel | 0,9 |
| SiOx** | 4,0 |
| Silicone Oil | 2,0 |
| Weißer Tee-Extrakt | 0,3 |
| Ginkgo-Extrakt | 1,0 |
| Buddleja davidii & Thymus vulgaris Extract | 0,01 |
| Citrus grandis Extrakt | 0,3 |
| Efeu-Extrakt | 0,7 |
| Sheabutter | 0,3 |
| β-Carotin | 0,1 |
| Silica & Titanium Dioxide & Iron Oxides | 0,2 |

** siehe Beispiel 1 *** RPF-Komplex 3 gemäß Anspruch 1 der WO 2004/105706 (mit folgenden Gewichtsanteilen, bezogen auf das Gesamtgewicht des RPF-Komplexes): Angelica archangelica-Wurzelextrakt, Pongamia pinnata-Samenextrakt, Camellia sinensis-Blattextrakt, Coffea arabica-Samenextrakt, Ethanol im Verhältnis 0,2 : 0,2 : 0,2 : 0,2 : 99,2.

Die Phase A wird bei Umgebungstemperatur (18-25°C) gemischt unter Rühren und mit Phase B neutralisiert. Unter Rühren werden nacheinander die Bestandteile der Phase C hinzugegeben bis zum Erreichen einer homogenen Gel-Mischung.

Beispiel 4 Vergleichstest

Es wurden Hautbiopsien mit Zigarettenteer behandelt und nach einer bestimmten Einwirkungszeit der Radikalschutzfaktor des Hauthomogenisats gemessen.

Die Hauptbiopsien wurden aus kosmetischen Operationen klinisch gesunder weiblicher Probanden im Alter von 35 bis 55 Jahren gewonnen. Nach Reinigung der Biopsien und Entfernung der Subcutis und Fascia wurden 1 cm$^2$ große Stücke geschnitten und für jeden Test vier gleiche Stücke genommen und daraus nach der Behandlung und Messung der Mittelwert festgestellt.

Mittels einer standardisierten Rauchmaschine wurde Teerkondensat der gleichen Zigarettenmarke (ohne Filter) gewonnen. Das Teerkondensat wurde in Alkohol gelöst mit einer Konzentration von 10 mg Teer / 1 ml Ethanol.

Die Haut wurde mit einer Basiscreme behandelt (2 mg/cm$^2$), die unterschiedliche Zusätze enthielt. Nach 4 h wurde die Haut mittels isotonischer NaCl-Lösung gereinigt.

Die nachfolgende Behandlungszeit mit 5 mg Teer betrug jeweils 4 h. Danach wurde der Teer mechanisch entfernt und die RPF-Messung erfolgte.

Die Basiscreme (Basis) bestand aus

Wasser (bis 100 %), Glycerin 2,0, Propylenglycol 3,0, Citronensäure 0,08, EDTA 0,1, Glycerylstearat 3,5, PEG-100 Stearate 1,0, Ceteareth-20 3,5, Siliconöl 3,0, Konservierungsmittel 0,5.

TEST A: Haut + Basis

TEST B: Haut + Basis + Teer

TEST C: Haut + Basis + 0,5 % Tee-Extrakt + Teer

TEST D: Haut + Basis + 1,5 % Ginkgo-Extrakt + Teer

TEST E: Haut + Basis + 0,8 % RPF-Komplex 1* + Teer

TEST F: Haut + Creme Beispiel 2 + Teer

*RPF-Komplex 1 = siehe Vermerk zu Beispiel 1.

Die Elektronenspinresonanz (ESR) - Messungen wurden mit einem Spektrometer ERS 300 mit Tomografie-Einheit ZZG1 (Privatinstitut Galenus GmbH, Deutschland) durchgeführt. Die Spektren wurden bei 100 kHz und die Aufnahmen bei 50 kHz Magnetfeldmodulation mit einer Modulationsamplitude von 0,15 mT bis 0,20 mT aufgezeichnet. Mikrowellenfrequenz 9,5 GHz; Mikrowellenleistung 20 mW; Testradikal: DPPH von Sigma, Deutschland.

Die ermittelten Faktoren wurden auf einen Wert 1 für unbehandelte Haut normalisiert (A = 1).

Für den Test B ergab sich ein Wert von 0,8, d.h. das Radikalschutzpotential war um 20 % herabgesetzt.

Für die Tests C, D und E ergaben sich Werte von 0,85 bzw. 0,90 bzw. 0,95, was eine Erhöhung des Radikalschutzpotentials um 6,3 % bzw. 12,5 % bzw. 18,8 % bedeutet gegenüber dem um 20 % herabgesetzten Wert.

Test F zeigte einen Wert von 1,15, was einer Erhöhung des Radikalschutzpotentials um 43,8 % entsprach gegenüber dem herabgesetzten Wert und wobei dieser Wert über dem Ausgangswert von 1 lag. Dies deutet auf einen Synergismus hin und demonstriert damit eindeutig die Überlegenheit des erfindungsgemäßen Produktes.

Eine Nachprüfung der Werte nach 24 Stunden zeigte bei Test F noch immer einen Wert von 1,03, während der Rückgang bei Test C, D und E deutlicher ausfiel mit 0,82 bzw. 0,86 bzw. 0,89. Auch aus dieser Langzeitwirkung wird die Überlegenheit des erfindungsgemäßen Produktes ersichtlich.

**Patentansprüche**

1. Kosmetisches Produkt zum Schutz der Haut gegen Umwelteinflüsse, **gekennzeichnet durch** folgende Bestandteile:

- ein Pflanzenextraktgemisch, bestehend aus Hedera helix-Extrakt, Buddleja davidii- Extrakt, Thymus vulgaris-Extrakt, Ginkgo biloba-Extrakt, Camellia sinensis-Extrakt und Citrus grandis-Extrakt;
- einen Sauerstoffträger, der aus einem flüssigen perfluorierten oder teilfluorierten Kohlenwasserstoff oder Kohlenwasserstoffgemisch, einem flüssigen Siliconpolymeren oder Siliconpolymerengemisch und einer Öl - oder Wasserbasis besteht, wobei dieses Trägersystem mit gasförmigem Sauerstoff beladen ist;
- einen Radikalfänger, der einen Komplex darstellt, bestehend aus Wasser, Gelbildner, Phospholipiden, Seidenraupen-Extrakt und einem **durch** Extraktion der Rinde von Quebracho blanco und nachfolgende enzymatische Hydrolyse gewonnenem Produkt oder der ein Gemisch von Pflanzenextrakten auf alkoholischer Basis darstellt, bestehend aus Angelica archangelica-Wurzelextrakt, Pongamia pinnata-Samenextrakt, Camellia sinensis-Blattextrakt, Coffea arabica-Samenextrakt, Ethanol im Verhältnis 0,2 : 0,2 : 0,2 : 0,2 : 99,2.
- ein teilchenförmiges, anorganisches oder organisches kosmetisch annehmbares Material mit einer Teilchengröße im Bereich von 1-25 μm;

und

kosmetische Hilfsstoffe, Trägerstoffe und Gemische davon.

2. Produkt nach Anspruch 1, **dadurch gekennzeichnet, dass** das teilchenförmige Material ein anorganisches Oxid oder Oxidgemisch ist.

3. Produkt nach Anspruch 1 oder 2, **dadurch gekennzeichnet, dass** die Teilchengröße des teilchenförmigen Materials im Bereich von 3-20 μm liegt.

4. Produkt nach einem der Ansprüche 1 bis 3, **dadurch gekennzeichnet, dass** das Produkt keinen Filmbildner enthält.

5. Produkt nach einem der Ansprüche 1 bis 4, **dadurch gekennzeichnet, dass** die Gesamtheit der Pflanzenextrakte nicht über 5 Gew.-% liegt.

6. Produkt nach einem der Ansprüche 1 bis 5, **dadurch gekennzeichnet, dass** der Anteil des Ginkgo-Extraktes im Bereich von 0,5 - 2,5 Gew.-% liegt.

7. Produkt nach einem der Ansprüche 1 bis 6, **dadurch gekennzeichnet, dass** der Anteil des Extraktgemisches Buddleja davidii und Thymus vulgaris im Bereich 0,01 - 3,0 Gew.-% liegt.

8. Produkt nach einem der Ansprüche 1 bis 7, **dadurch gekennzeichnet, dass** der Anteil des Hedera helix-Extraktes im Bereich 0,01 - 3,0 Gew.-% liegt.

9. Produkt nach einem der Ansprüche 1 bis 8, **dadurch gekennzeichnet, dass** der Anteil des Weißtee-Extraktes im Bereich 0,1 - 1,0 Gew.-% liegt.

10. Produkt nach einem der Ansprüche 1 bis 9, **dadurch gekennzeichnet, dass** der Anteil des Citrus grandis-Extraktes im Bereich 0,1 - 2,0 Gew.-% liegt.

11. Verwendung des kosmetischen Produktes nach den Ansprüchen 1 bis 10 zum Schutz der Haut gegen verschmutzte Luft, insbesondere gegen Rauch, Ruß und/oder Staub.

12. Verwendung des kosmetischen Produktes nach Anspruch 11 zum Schutz der Haut gegen Zigarettenrauch, Dieselrußpartikel und/oder Gemischen davon.

**Claims**

1. A cosmetic product for protecting the skin against environmental influences, **characterized by** the following components:

   - a mixture of plant extracts consisting of Hedera helix extract, Buddleja davidii extract, Thymus vulgaris extract, Ginkgo biloba extract, Camellia sinensis extract and Citrus grandis extract;
   - an oxygen carrier which consists of a liquid perfluorinated or partially fluorinated hydrocarbon or hydrocarbon mixture, a liquid silicone polymer or silicone polymer mixture and an oil or water base, said carrier system being loaded with gaseous oxygen;
   - a free-radical scavenger which is a complex consisting of water, gelling agent, phospholipids, silkworm extract and a product obtained by extraction of the bark of Quebracho blanco and subsequent enzymatic hydrolysis or which is a mixture of alcohol-based plant extracts consisting of Angelica archangelica root extract, Pongamia pinnata seed extract, Camellia sinensis leaf extract, Coffea arabica seed extract, ethanol at a ratio of 0.2 : 0.2 : 0.2 : 0:2 : 99.2;
   - a particulate, inorganic or organic, cosmetically acceptable material having a particle size in the range of 1-25 μm; and
   - cosmetic adjuvants, carrier substances and mixtures thereof.

2. The product according to claim 1, **characterized in that** the particulate material is an inorganic oxide or oxide mixture.

3. The product according to any of claims 1 or 2, **characterized in that** the particle size of the particulate material is in the range of from 3 to 20 μm.

4. The product according to any of claims 1 to 3, **characterized in that** the product does not include any film-forming agent.

5. The product according to any of claims 1 to 4, **characterized in that** the total of the plant extracts is no more than 5% by weight.

6. The product according to any of claims 1 to 5, **characterized in that** the share of Ginkgo extract is in the range of from 0.5 to 2.5 % by weight.

7. The product according to any of claims 1 to 6, **characterized in that** the share of the Buddleja davidii and Thymus vulgaris extract mixture is in the range of from 0.01 to 3.0% by weight.

8. The product according to any of claims 1 to 7, **characterized in that** the share of Hedera helix extract is in the range of from 0.01 to 3.0% by weight.

9. The product according to any of claims 1 to 8, **characterized in that** the share of white tea extract is in the range of from 0.1 to 1.0% by weight.

10. The product according to any of claims 1 to 9, **characterized in that** the share of Citrus grandis extract is in the range of from 0.1 to 2.0% by weight.

11. Use of the cosmetic product according to any of claims 1 to 10 for protecting the skin against polluted air, especially against smoke, soot and/or dust.

12. The use of the cosmetic product according to claim 11 for protecting the skin against cigarette smoke, diesel soot particles and/or mixtures thereof.

**Revendications**

1. Produit cosmétique destiné à assurer la protection de la peau contre les influences de l'environnement, **caractérisé par** les composants suivants :

  - un mélange d'extraits végétaux, constitué d'extrait d'Hedera helix, d'extrait de Buddleja davidii, d'extrait de Thymus vulgaris, d'extrait de Ginkgo biloba, d'extrait de Camellia sinensis et d'extrait de Citrus grandis ;
  - un oxydant constitué d'un hydrocarbure ou d'un mélange d'hydrocarbures perfluoré ou partiellement fluoré, d'un polymère de silicone ou d'un mélange de polymères de silicone liquide et d'une base huileuse ou aqueuse, ce système porteur étant chargé en oxygène gazeux ;
  - un anti-radicaux libres représentant un complexe constitué d'eau, d'un agent gélifiant, de phospholipides, d'extrait de vers à soie et d'un produit obtenu par extraction de l'écorce de Quebracho blanco et hydrolyse enzymatique suivante ou représentant un mélange d'extraits végétaux sur base alcoolique, constitué d'extrait de racines d'Angelica archangelica, d'extrait de graines de Pongamia pinnata, d'extrait de feuilles de Camellia sinensis, d'extrait de graines de Coffea arabica, d'éthanol dans un rapport de 0,2 : 0,2 : 0,2 : 0,2 : 99,2 ;
  - une matière anorganique ou organique en particules acceptable en cosmétique, d'une grandeur de particules de l'ordre de 1 à 25 μm ;

  et
  des adjuvants et supports cosmétiques et des mélanges de ces derniers.

2. Produit selon la revendication 1, **caractérisé en ce que** la matière en particules est un oxyde anorganique ou un mélange d'oxydes.

3. Produit selon la revendication 1 ou la revendication 2, **caractérisé en ce que** la grandeur des particules de la matière en particules est de l'ordre de 3 à 20 μm.

4. Produit selon l'une quelconque des revendications 1 à 3, **caractérisé en ce que** le produit ne contient aucun agent filmogène.

5. Produit selon l'une quelconque des revendications 1 à 4, **caractérisé en ce que** la totalité des extraits végétaux n'est pas supérieure à 5 % en poids.

6. Produit selon l'une quelconque des revendications 1 à 5, **caractérisé en ce que** la part de l'extrait de Ginkgo-est de l'ordre de 0,5 à 2,5 % en poids.

7. Produit selon l'une quelconque des revendications 1 à 6, **caractérisé en ce que** la part du mélange d'extraits de Buddleia davidii et de Thymus vulgaris est de l'ordre de 0,01 à 3,0 % en poids.

8. Produit selon l'une quelconque des revendications 1 à 7, **caractérisé en ce que** la part de l'extrait d'Hedera helix est de l'ordre de 0,01 à 3,0 % en poids.

9. Produit selon l'une quelconque des revendications 1 à 8, **caractérisé en ce que** la part de l'extrait de thé blanc est de l'ordre de 0,1 à -1,0 % en poids.

10. Produit selon l'une quelconque des revendications 1 à 9, **caractérisé en ce que** la part de l'extrait de Citrus grandis est de l'ordre de 0,1 à 2,0 % en poids.

11. Utilisation du produit cosmétique selon les revendications 1 à 10 pour assurer la protection de la peau contre les pollutions atmosphériques, notamment contre la fumée, la suie et/ou la poussière.

12. Utilisation du produit cosmétique selon la revendication 11, pour assurer la protection de la peau contre la fumée de cigarettes, les particules de suie de diesel et/ou des mélanges de ces derniers.

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- US 5683684 A
- US 2004047823 A
- US 2004013696 A
- US 20020168328 A
- WO 0059465 A
- WO 2006111666 A
- EP 1498113 A
- FR 2768925 A

- FR 2831444 A
- EP 968709 A
- US 2007003536 A1
- WO 9966881 A
- WO 0126617 A
- WO 2004105704 A
- WO 2004105706 A

**In der Beschreibung aufgeführte Nicht-Patentliteratur**

- Modern Challenges To The Cosmetic Formulation. **HERRLING ; GROTH ; FUCHS ; ZASTROW.** Conference Materials. Verlag f. chem. Ind, 1997, 150-155